# EUROPÄISCHE PATENTSCHRIFT

(11) **EP 3 503 865 B1**
(45) Veröffentlichungstag und Bekanntmachung des Hinweises auf die Patenterteilung: **09.04.2025**
(21) Anmeldenummer: 15834722.9
(22) Anmeldetag: 30.12.2015
(51) Int. Cl.: A61K 8/25, A61P 17/14, A61K 8/19, A61K 8/67, A61Q 7/00, A61K 8/44, A61K 8/27, A23L 33/155, A23L 33/16, A23L 33/175, A61K 31/122, A61K 31/197, A61K 31/198, A61K 31/20, A61K 31/375, A61K 31/4188, A61K 31/465, A61K 31/519, A61K 31/593, A61K 31/714

(54) **HAARWUCHSPRÄPARAT ENTHALTEND L-CYSTEIN**
HAIR RESTORER PREPARATION COMPRISING L-CYSTEINE
PRÉPARATION POUR LA REPOUSSE DES CHEVEUX COMPRENANT DE LA L-CYSTÉINE

(30) Priorität: 30.12.2014 CH 20572014
(43) Veröffentlichungstag der Anmeldung: 03.07.2019
(73) Patentinhaber: SH Power AG, 6300 Zug (CH)
(72) Erfinder: Wietlisbach, Simone, 8834 Schindellegi (CH)
(86) Internationale Anmeldenummer: PCT/IB2015/060047
(87) Internationale Veröffentlichungsnummer: WO 2016/108195

(56) Entgegenhaltungen:
- EP-A2- 0 799 578
- EP-A2- 0 799 578
- WO-A1-2005/079857
- WO-A1-2005/079857
- WO-A2-91/11117
- WO-A2-91/11117
- DE-U1- 9 412 374
- DE-U1- 9 412 374
- US-A- 6 149 933
- US-A- 6 149 933
- DATABASE GNPD MINTEL; March 2009 (2009-03-01), WELLNESS & NUTRITION INDIA: "Condition specific nutritional supplement for hair, skin & nails"
- DATABASE GNPD [online] MINTEL; March 2009 (2009-03-01), WELLNESS & NUTRITION INDIA: "Condition specific nutritional supplement for hair, skin & nails", XP002755478, Database accession no. 1046703
- FIT HAIR, INC.: "Hair Fitness (Hair fitness Nutritional Supplement)", 2009, XP002755479, Retrieved from the Internet <URL:http://www.healthandbodyfitness.com/supplement.htm> [retrieved on 20160314]

## Beschreibung

Die vorliegende Erfindung betrifft ein Haarwuchspräparat, das nicht für die äußere Applikation auf der Kopfhaut, sondern für die orale Einnahme gedacht ist.

Es sind Haarwuchspräparate zur oralen Einnahme bekannt, welche die Grundidee aufgreifen, dass eine verbesserte Versorgung des Organismus und des Metabolismus mit den nötigen Säuren, Vitaminen, Mineralstoffen, Spurenelementen usw. einen positiven Einfluss auf den Haarwuchs ausüben kann.

So zum Beispiel ist seit längerer Zeit Priorin^{®} auf dem Markt, ein Haarwuchspräparat, das L-Cystein enthält, eine schwefelhaltige proteinogene Aminosäure, sowie einen Gesamtextrakt aus Hirsefrüchten und Calciumpantothenat. Der Nachteil dieses Haarwuchspräparates ist jedoch, dass es nur ein sehr schmales Spektrum der in ihrer Gesamtheit notwendigen Stoffe abdeckt, die alle für einen gesunden Haarwuchs mitverantwortlich sein können. Des Weiteren ist die Zusammensetzung zu undifferenziert auf die individuellen Voraussetzungen abgestimmt.

Ein weiteres Haarwuchspräparat, das ebenfalls zur oralen Einnahme entwickelt wurde, ist beispielsweise amitamin^{®} Hair Plus. Dieses Haarwuchspräparat will ein größeres Spektrum der notwendigen bzw. förderlichen Stoffe abdecken, indem es neben den bereits genannten Substanzen auch noch Hefe, Biotin, Vitamin E, Aminosäuren, Eisen, Zink u.a. enthält. Aber auch dieses Präparat ist nicht kundenspezifisch abgestimmt.

Es stellt generell vor allem aus dem Blickwinkel einer Wirtschaftlichkeits-Erfolgs-Relation einen Nachteil dar, wenn undifferenzierte "Rundumkeulen" auf den Markt geworfen werden, die zu einem hohen Prozentsatz nicht wirken, und wenn sie wirken, es eher per Zufall tun.

Die Undifferenziertheit solcher Haarwuchspräparate "ab Werk", in Form von Standard- oder Monopräparaten, stellt des Weiteren nicht nur hinsichtlich des Vorhandenseins der optimalerweise erforderlichen - unter Umständen recht teuren - Substanzen oder Substanzenzusammensetzungen einen Nachteil dar, sondern auch hinsichtlich deren Dosierung. Diese sind nämlich in einer Höhe gehalten, die noch generell vertretbar und entsprechend gering gehalten ist, obwohl die Kundin oder der Kunde ganz insbesondere auf eine bestimmte Substanz oder Substanzenzusammensetzung in relativ erhöhter Dosis angewiesen wäre, im Verhältnis zu den restlichen Substanzen oder Substanzenzusammensetzungen.

Weiterhin nachteilig sind Haarwuchspräparate, die hormonell wirkende Substanzen oder Substanzenzusammensetzungen enthalten. So zum Beispiel ist es erforderlich, bei einer einmal begonnenen Einnahme des beispielhaften Wirkstoffes Finasterid aus dem Medikament Propecia^{®}, diese Einnahme auch ein Leben lang beizubehalten. Sonst verschlimmert sich die Situation über das ursprüngliche Ausgangsstadium hinaus. Des Weiteren ist dieses Haarwuchspräparat für Frauen nicht geeignet und greift mit erheblichen negativen Nebenwirkungen in den hormonellen Haushalt ein, indem der Dihydrotestosteronspiegel (DHT-Spiegel) langfristig unterdrückt wird.

Des Weiteren gibt es Pantostin mit dem Wirkstoff Alfastradiol, mittels dessen nicht hormonell, sondern quasi medikamentös bzw. chemisch der Rückbildung der Haarwurzeln entgegenzuwirken versucht wird.

Pantovigar N wird ebenfalls oral eingenommen und versucht mittels B-Vitaminen und Cystein die Nährstoffversorgung so zu verbessern, dass der Aufbau von Haarkeratin gefördert wird.

Des Weiteren gibt es von den Oxford Biolabs entwickelte und unter der Bezeichnung TRX2^{®} vertriebene Kapseln, als ein zum Patent angemeldetes Mittel gegen Alopezie. Dieses Patent ist zum Zeitpunkt des Verfassens dieser vorliegenden Patentanmeldung noch nicht veröffentlicht und beschreibt eine Kombination kraftvoller metabolischer Stimulanzien (Kalium, Nikotinsäure und BCAA = Branched-Chain Amino Acids = verzweigtkettige Aminosäuren, also Valin, Leucin und Isoleucin) mit einer natürlichen Energie anregenden Substanz (Carnipur^{™} tartrat). Diese Kombination soll wiederum den Haarausfall stoppen und den Haarwuchs auf molekularer Ebene fördern. Jedoch auch dieses Mittel ist nicht auf die Kundin oder den Kunden individuell "maßgeschneidert".

Das US-Patent US 6,149,933, erteilt am 21.11.2000, offenbart ein Nahrungsergänzungsmittel für die Förderung gesunden Haares und Pigment-Wiederherstellung. Es wird kein Haarwuchspräparat mit therapeutischer Wirkung beansprucht, sondern ein Nahrungsergänzungsmittel, das der Ergrauung entgegenwirken soll. Das beschriebene Nahrungsergänzungsmittel enthält im Wesentlichen ein Kupfersalz, Para-Aminobenzoesäure oder Salze hiervon, Pantothensäure oder Salze hiervon und Vitamin B6. Es enthält kein L-Cystein, kein Zink, kein Silicium und auch kein Cholecalciferol bzw. Vitamin D3.

Die internationale Veröffentlichungsschrift WO 2005/079857 A1 vom 01.09.2005 beschreibt ein Verfahren zur Erhöhung von Nährstoff-Bioverfügbarkeiten durch Anwendung von Polysacchariden, wie Galaktomannane, Glucomannane und dergleichen zur Einschleusung in den menschlichen oder tierischen Stoffwechsel von Wirkstoffen, wie zum Beispiel des menschlichen Wachstumshormons HGH.

Ein von der Firma Wellness & Nutrition India 2009 vertriebenes Nahrungsergänzungsmittel «Condition Specific Nutrition For Hair, Skin & Nails» beansprucht für sich, aufgrund der Beinhaltung von Zink, Vitamin E und Omega 3, wichtig für Haut, Haare und Nägel zu sein. Das Nahrungsergänzungsmittel kann noch weitere Nährstoffe enthalten, offenbart aber kein Silicium und Cholecalciferol bzw. Vitamin D3, sowie keine «Maßschneiderung» auf die exakten und ermittelten Nährstoffbedürfnisse eines Kunden.

Ein weiteres Nahrungsergänzungsmittel «Hair Fitness^{®} Nutritional Supplement» verspricht dickes, seidiges und jung aussehendes Haar, enthält aber ebenfalls kein Silicium und Cholecalciferol bzw. Vitamin D3 und sieht auch keine individuell angepassten Tagesdosen vor.

Die Aufgabe der vorliegenden Erfindung ist, unter Vermeidung der oben aufgezeigten Nachteile ein Haarwuchspräparat zu stellen, das eine nochmals verbesserte Grundzusammensetzung aufweist und auf individuelle Bedürfnisse einer Kundin oder eines Kunden variier- und oder adaptierbar ist und generell in seinen Eigenschaften und seiner Anwendung optimiert ist.

Der Schutzumfang des Patents wird durch die beigefügten Ansprüche definiert. Die Lösung der Aufgabe besteht zunächst in der erfindungsgemäßen Zusammensetzung eines Haarwuchspräparates in einer Basisvariante, die folgende Grundsubstanzen oder Grund-Substanzenzusammensetzungen umfasst:
- L-Cystein
- Zink
- Kupfer
- Calcium
- Magnesium
- Silicium
- Vitamin D3.

Wie bereits eingangs erwähnt, ist L-Cystein eine schwefelhaltige proteinogene Aminosäure und Baustein des Haarkeratins und somit ein wichtiges Strukturelement für Haare und Nägel. Dank des Schwefelgehaltes kann L-Cystein Schwermetall-Ionen an sich binden, die wiederum Haarausfall verursachen können. L-Cystein ist zusammen mit anderen Aminosäuren (Glutamin, Glycin) am Aufbau von Glutathion beteiligt, das eines der wichtigsten Antioxidanzien im menschlichen Körper ist und der Verwertung von Vitamin C und E zuträglich ist. Glutathion hemmt außerdem entzündliche Reaktionen und regt die Produktion von weißen Blutkörperchen und Leukotrienen an. Die menschliche Leber kann körpereigenes L-Cystein synthetisieren, es muss jedoch auch über die Nahrung aufgenommen werden, mit einem täglichen Bedarf von etwa 1'400 mg. Dieser Bedarf ist oft nicht gedeckt, weil bestimmte Erkrankungen wie etwa Grauer Star oder Arthritis oder sonstige chronische Krankheiten oder aber auch Diäten oder Stress oder körperliche Anstrengung ihn erhöhen. Es kommt erschwerend hinzu, dass bei Zubereitungsweisen, in denen sich die Lebensmittel lange im Wasser befinden oder gekocht werden, das gut wasserlösliche L-Cystein ausgewaschen wird.

Das Spurenelement Zink hingegen ist im menschlichen Organismus maßgeblich an der Zellteilung, also generell dem Wachstum und der Zell- und Hautregeneration, an der Insulinspeicherung, der Eiweißsynthese, der Spermienproduktion und dem Immunsystem beteiligt. Zink ist generell am Hormon-Metabolismus beteiligt, wirkt positiv auf das Zentralnervensystem bei Stress, reguliert den Säure-Basen-Haushalt und hilft, Schwermetalle auszuleiten. Ein Zinkmangel kann unter anderem zu vermehrtem Haarausfall, schlechter und zu Entzündungen neigender Haut und zu einer Schwächung des Immunsystems führen. Zinkmangel ist besonders bei vegetarischen Ernährungsformen ein Problem, da Zink vor allem in Fleisch, Käse, Milch und Eiern enthalten ist. Das in Getreideerzeugnissen enthaltene Zink ist bei eiweißarmer Ernährung weniger gut aus dem Darm aufnehmbar. Es muss bei einer Zinkgabe darauf geachtet werden, dass bei einer gleichzeitigen Gabe von Eisenpräparaten die Zinkaufnahme im Darm verschlechtert ist.

Die oben beschriebene Grundzusammensetzung eines erfindungsgemäßen Haarwuchspräparates umfasst des Weiteren Kupfer. Das Spurenelement Kupfer ist an dem Aufbau von Kollagen beteiligt und sorgt somit für eine Straffung von Haut-, Binde-, Nervengewebe und der Blutgefäße. Des Weiteren ist das Spurenelement Kupfer beteiligt an der Bildung von Melanin, dem Farbstoff für Haare, Haut und Augen. Graue Haare können einen Kupfermangel als Ursache haben. Weiterhin ist Kupfer maßgeblich beteiligt an der Bildung von körpereigenen Antioxidanzien, die den Alterungsprozess verzögern.

Calcium wiederum ist für den menschlichen Körper kein Spurenelement mehr, sondern ein Mengenelement, d.h. mit mehr als 50 mg pro kg Körpergewicht. Mit einem Körperbestand von 1-1.1 kg ist Calcium der mengenmäßig am stärksten vertretene Mineralstoff im menschlichen Organismus. 99% hiervon befinden sich in Knochen und Zähnen, aber das Calcium ist im Körper auch wesentlich an der Erregung der Muskeln und Nerven, dem Glykogen-und Aminosäuren-Stoffwechsel, der Zellteilung sowie an der Aktivierung einiger Enzyme und Hormone beteiligt. Die empfohlene Tageszufuhr für einen erwachsenen Mann liegt sehr grob bei etwa 1'000 mg. Es ist zu beachten, dass gerade bei eiweißreicher Kost ein erhöhter Calciumbedarf besteht.

Weiterhin gehört Magnesium zu den essenziellen Stoffen, weil es für alle Organismen unentbehrlich ist. Es muss dem Körper täglich in ausreichender Menge (ca. 300 mg) über die Nahrung und Trinkwasser zugeführt werden und die Resorption findet im oberen Dünndarm statt. Der Körper eines Erwachsenen enthält etwa 20 g Magnesium, das an ca. 300 Enyzmreaktionen als Enzymbestandteil oder Coenzym beteiligt ist. Zudem beeinflussen freie Magnesiumionen das Potenzial an den Zellmembranen und fungieren als second messenger im Immunsystem. Sie stabilisieren das Ruhepotenzial von erregbaren Muskel- und Nervenzellen und der Zellen des autonomen Nervensystems. Magnesium ist ein Muskel- und Bindegewebe-Relaxans. Magnesiummangel löst Ruhelosigkeit, Nervosität, Reizbarkeit, Kopfschmerzen, Konzentrationsmangel, Müdigkeit, allgemeines Schwächegefühl, Herzrhythmusstörungen, Muskelkrämpfe und natürlich auch mindestens einen so schlechten Allgemeinzustand in der Nährstoffversorgung aus, dass das Haarwachstum in Mitleidenschaft gezogen wird.

Silicium ist ein Hauptelement der Knochen bildenden Zellen, aber auch ein wichtiges Strukturelement für Knorpel, Binde- und Knochengewebe, Haut, Haare und Fingernägel. Bei Haarausfall wurden niedrige Haar-Siliciumwerte beobachtet. Chronische Belastungen mit Aluminium scheinen die Bioverfügbarkeit vom Silicium zu beeinträchtigen.

Vitamin D3 bzw. Cholecalciferol ist im menschlichen Organismus unter anderem dafür erforderlich, dass er größere Mengen von Calcium aufnehmen kann, indem es durch Bildung eines Proteincarriers den Transport des Calciums durch die Darmwand gewährleistet. Vitamin D3 ist das einzige Vitamin, bei dem die biologisch aktive Form ein Hormon ist, welches im Organismus eine Breitbandfunktion einnimmt. Es ist u.v.a. am Knochen- und Zähneaufbau beteiligt, aber auch am Immunsystem, indem es die Funktion der weißen Blutkörperchen gegen Infektionen unterstützt. Durch den endemischen Lichtmangel der modernen Gesellschaften findet in der Haut eine nur unzureichende körpereigene Synthese von Vitamin D3 statt und somit gab die Deutsche Gesellschaft für Ernährung (DGE) einen Richtwert für die tägliche Vitamin-D3-Menge von 20 µg für einen Erwachsenen aus, die alimentär abgedeckt werden soll. Es kommt erschwerend hinzu, dass nicht nur Sonnenschutzcremen, sondern so gut wie alle heutigen Tagescremen UV-B- und UV-A-Filter enthalten.

Die Dosierung dieser Grundsubstanzen oder Grund-Substanzenzusammensetzungen ist an die individuellen organischen Nährstoff-Versorgungs-Voraussetzungen der Kundin oder des Kunden variier- und adaptierbar, in Funktion der Ergebnisse von individuellen Tests und Messungen, aber auch je nach Auswertungsergebnis von Fragebögen.

Die vorliegende Erfindung beruht auf den folgenden Grundideen: Selbstverständlich können den Kopfhaaren eines Menschen gewisse Körperfunktionen oder ein organischer "Zweck" zugewiesen werden, diese bzw. dieser sind jedoch für den Organismus nicht von vitaler Bedeutung. Beispielsweise bei Todesgefahr durch Erfrierung wird die Blutversorgung der Extremitäten oder der für die Vitalfunktionen des Organismus weniger wichtigen Organe peu à peu eingestellt, und nur zuallerletzt die Blutversorgung von Herz und Gehirn. So ähnlich geht der Organismus mit einem persistierenden Nährstoffmangel um und es erfolgt eine prioritäre Zuteilung der vorhandenen Nährstoffe zuerst zu den lebenswichtigen Organen und Körperteilen und erst in einer niedrigeren oder gar letzten Priorität dem Haarwuchs.

Eine zweite Grundidee ist, dass weniger der Haarausfall per se genetisch vererbt wird, sondern Lebensgewohnheiten und die Fähigkeit, bestimmte Nährstoffe besser oder schlechter zu resorbieren resp. aufzunehmen resp. zu verwerten. Des Weiteren liegt einem erfindungsgemäß adaptierbaren Haarwuchspräparat die Erkenntnis zugrunde, dass ca. 80% der Männer von Haarausfall betroffen sind und nur ca. 38% der Frauen. Somit ist es geboten, zumindest hinsichtlich des Geschlechtes eine Adaption des Haarwuchspräparates an die unterschiedlichen Voraussetzungen der beiden Geschlechter vorzunehmen, besser noch an weitere individuelle Voraussetzungen.

Weiterhin liegt einem erfindungsgemäßen Haarwuchspräparat die Erkenntnis zugrunde, dass der Vitamin- und Nährstoffgehalt der Nahrungsmittel in unseren Breitengraden um ca. 30% zurückgegangen ist und gleichzeitig stressigere und hektischere Lebensweise einer erhöhten Zufuhr von Vitalstoffen wie Vitaminen, Spurenelementen, Mineralstoffen, Aminosäuren und Quasivitaminen bedarf.

Einem erfindungsgemäßen Haarwuchspräparat liegen des Weiteren Erkenntnisse der orthomolekularen Medizin zugrunde. Danach findet eine im Organismus optimale Verstoffwechslung erst statt, wenn die hohe Anzahl der verschiedenen Vitalstoffe in unterschiedlicher und aufeinander abgestimmter Dosierung gewährleistet ist. Bereits ein einziger Mangel unter den mehr als zwei Dutzend geforderten Mikronährstoffen kann die gesamte Verstoffwechslung bremsen oder gar blockieren. Demzufolge begnügt sich die Zusammensetzung eines erfindungsgemäßen Haarwuchspräparates nicht nur mit einzelnen Substanzen oder Substanzenzusammensetzungen und die in den üblich vorsichtig empfohlenen Mindestdosierungen, sondern umfasst vorzugsweise viele oder alle vom Körper benötigten Substanzen und ohne Weiteres die eine oder andere in sehr hoher Dosierung.

Eine weitere Ausgestaltungsvariante eines weiterhin erfindungsgemäßen Haarwuchspräparates umfasst über die in dem Absatz [0016] aufgezählten Grundsubstanzen oder Grund-Substanzenzusammensetzungen hinaus noch Vitamin E. Vitamin E ist ein Sammelbegriff für lipidlösliche Substanzen mit antioxidativer Wirkung, die am häufigsten in Form von Tocopherolen und Tocotrienolen vorkommen. Vitamin E hilft dem Immunsystem, Oxydationseinflüsse aus der Umwelt und toxische Stoffe auszuleiten und Bakterien zu zerstören. Es ist des Weiteren beobachtet worden, dass bei Kost mit erhöhter Anzahl von mehrfach ungesättigten Fettsäuren der Bedarf an Vitamin E steigt. Die Zusammensetzung eines erfindungsgemäßen Haarwuchspräparates kann nicht zuletzt deshalb Vitamin E umfassen, weil erkannt wurde, dass Vitamin E die Durchblutung der Kopfhaut fördert und somit die Verankerung der Haare festigt. Des Weiteren wird Vitamin E für die Synthese verschiedener für die Haare wichtiger Nährstoffe benötigt.

Weiterhin umfasst eine weitere Ausgestaltungsvariante eines erfindungsgemäßen Haarwuchspräparates über die in Absatz [0016] und/oder in den Absätzen [0016] und [0029] aufgezählten Grundsubstanzen oder Grund-Substanzenzusammensetzungen hinaus noch Vitamin C bzw. Ascorbinsäure. Dieses beruht auf den Erkenntnissen, dass Vitamin C beim Entgiften hilft, z.B. von Haarausfall verursachenden Schwermetallen, ein wirksames Antioxidans ("Radikalfänger") ist, als Stressbewältiger fungiert und somit mehr Nährstoffe für das Haarwachstum zur Verfügung stehen. Des Weiteren ist Vitamin C wesentlich an der Kollagenherstellung beteiligt und verbessert darüber hinaus die Struktur des Haares.

Eine weitere Ausgestaltungsvariante eines weiterhin erfindungsgemäßen Haarwuchspräparates umfasst über die bisher offenbarten Grundsubstanzen oder Grund-Substanzenzusammensetzungen hinaus auch Biotin bzw. Vitamin H bzw. Vitamin B7. Dieses beruht auf der Erkenntnis, dass Biotin wesentlich an der Stoffwechselaktivierung und dem Zellwachstum beteiligt ist und ein Mangel davon unter anderem zu vermehrtem Haarausfall führen kann.

Eine weitere Zusammensetzungsvariante eines erfindungsgemäßen Haarwuchspräparates umfasst auch L-Methionin, eine essenzielle, wie L-Cystein ebenfalls schwefelhaltige proteinogene Aminosäure, aus der das L-Cystein gebildet wird. Das L-Methionin ist wesentlich am Aufbau von Prokollagen beteiligt und somit wichtig für die Haarbildung.

Eine weiterhin bevorzugte Zusammensetzungsvariante eines erfindungsgemäßen Haarwuchspräparates umfasst auch Pantothensäure, trivial auch Vitamin B5 genannt. Es ist das wasserlösliche Vitamin aus der Reihe der B-Vitamine und ist beteiligt an dem Auf- und Abbau von Kohlenhydraten, Fetten, Aminosäuren und an der Synthese von Cholesterin, das wiederum für die Bildung der Steroidhormone gebraucht wird. Ein Mangel kann zu Müdigkeit, Schlaflosigkeit, Depressionen, tauben und schmerzenden Muskeln, Anämie, Immunschwäche und Magenschmerzen führen und somit zu einem so schlechten Allgemeinzustand, dass dem Haarwuchs die erforderliche Nährstoffzufuhr abgezogen wird.

Eine weiterhin bevorzugte Ausgestaltungsvariante eines erfindungsgemäßen Haarwuchspräparates umfasst neben den offenbarten Grundsubstanzen oder den Grundsubstanzen zuzüglich einer oder zuzüglich einer Kombination der optionalen Substanzen oder zuzüglich aller optionalen Substanzen auch Eisen. Eisen ist ein essenzielles Spurenelement, das wesentlich an der Blutbildung und dem Sauerstofftransport, der Sauerstoffspeicherung und der Elektronenübertragung beteiligt ist. Weiter ist Eisen Bestandteil von Eisen-Schwefel-Komplexen in vielen Enzymen. Es ist erkannt worden, dass nicht nur bei Frauen wegen der Menstruation und Geburten oder bei Sportlern ein Eisenmangel häufig ist, sondern generell eine Unterversorgung herrscht. Bei der Zusammensetzung eines erfindungsgemäßen Haarwuchspräparates mit Eisen wird darauf geachtet, dass eine gleichzeitige Einnahme von Vitamin C die Resorptionsquote von Eisen deutlich erhöht, Milchprodukte, Kaffee oder schwarzer Tee sie jedoch hemmen.

Eine weiterhin bevorzugte Ausgestaltungsvariante eines erfindungsgemäßen Haarwuchspräparates umfasst neben den offenbarten Grundsubstanzen oder den Grundsubstanzen zuzüglich einer oder zuzüglich einer Kombination der optionalen Substanzen oder zuzüglich aller optionalen Substanzen auch Vitamin B₆ bzw. die drei Vorstufen des aktivierten Vitamins Pyridoxalphosphat, nämlich Pyridoxin, Pyridoxal und Pyridoxamin. Vitamin B₆ ist elementar für den Aminosäuren-Stoffwechsel und wirkt als Coenzym in etwa 100 enzymatischen Reaktionen. Mangelerscheinungen von Vitamin B₆ äußern sich in Appetitverlust, Durchfall, Erbrechen, Dermatitis, Wachstumsstörungen, Anämien, Nervendegeneration, Krampfzuständen in unregelmäßigen Intervallen und Angststörungen. Der Bedarf von Vitamin B₆ ist direkt proportional mit der Eiweißzunahme und wird von der Deutschen Gesellschaft für Ernährung (DGE) mit einer Tagesdosis von 1.6 mg für Frauen und 1.8 mg für Männer empfohlen.

Eine weiterhin bevorzugte Ausgestaltungsvariante eines erfindungsgemäßen Haarwuchspräparates umfasst neben den offenbarten Grundsubstanzen oder den Grundsubstanzen zuzüglich einer oder zuzüglich einer Kombination der optionalen Substanzen oder zuzüglich aller optionalen Substanzen auch Vitamin B₁₂ bzw. Cobalamin, benannt nach seinem Zentralatom Cobalt. Vitamin B₁₂ ist im menschlichen Organismus wichtig für die Zellteilung und Blutbildung sowie die Funktion des Nervensystems. Der menschliche Organismus ist nicht in der Lage, Vitamin B₁₂ selbst herzustellen und muss es somit über die Nahrung zugeführt bekommen. Dieses ist vor allem über den Konsum von Fleisch und Innereien gewährleistet und muss bei vegetarischer Kost vermehrt über den Konsum von Milchprodukten und Eiern oder Nori-Algen erfolgen. Bei einem Mangel von Vitamin B₁₂ kann es zu Erkrankungen des Blutbildes und zu Myelitis kommen, einer Entzündung des Rücken- oder Knochenmarks. In den letzten Jahren mehrten sich zudem die Hinweise auf einen möglichen Zusammenhang zwischen einem Vitamin-B₁₂-Mangel und anderen Krankheitsbildern wie z.B. Demenz und Neuropathien. Grundsätzlich sind niedrige Vitamin-B₁₂-Konzentrationen im Blutserum bei älteren Menschen häufiger zu beobachten. Erste Anzeichen einer Vitamin-B₁₂-Unterversorgung bei erwachsenen Personen können Kribbeln und Kältegefühl in Händen und Füßen, Erschöpfung und Schwächegefühl, Konzentrationsstörungen und sogar Psychosen sein. Der tägliche Mindestbedarf für einen Erwachsenen beträgt ca. 3.0 µg und bei Schwangeren und Stillenden 3.5-4.0 µg.

Eine weiterhin bevorzugte Ausgestaltungsvariante eines erfindungsgemäßen Haarwuchspräparates umfasst neben den offenbarten Grundsubstanzen oder den Grundsubstanzen zuzüglich einer oder zuzüglich einer Kombination der optionalen Substanzen oder zuzüglich aller optionalen Substanzen auch Selen. Selen ist für alle Lebensformen ein essenzielles Spurenelement, das unter anderem wegen seiner hohen Reaktivität mit Sauerstoff die Zellmembranen vor oxidativer Zerstörung schützt. Des Weiteren spielt Selen und seine Verbindungen eine wichtige Rolle bei der Produktion der Schilddrüsenhormone. Allerdings ist ein positiver Wirkungseffekt von einer Selengabe nur in einem sehr schmalen und exakten Dosierungsbereich zu beobachten. Es gibt Studien, die den Verdacht der Verursachung von Typ 2 Diabetes durch eine Gabe von 200 µg Selen pro Tag belegen. Und bei Beschäftigten in der Elektronik-, Glas- und Farbenindustrie, die Tagesdosierungen von mehr als 400 µg ausgesetzt sind, treten u.a. Vergiftungserscheinungen auf wie Übelkeit und Erbrechen, Nagelveränderungen, periphere Neuropathie, Erschöpfung und ausgerechnet Haarverlust.

Eine weitere Zusammensetzungsvariante eines erfindungsgemäßen Haarwuchspräparates umfasst auch Niacin bzw. Nicotinsäure, die ein weiteres Vitamin aus dem B-Komplex ist. Sie ist ein kristalliner Feststoff, der sich in allen lebenden Zellen befindet und in der Leber gespeichert wird. Sie ist von zentraler Bedeutung für den Stoffwechsel von Eiweißen, Fetten und Kohlenhydraten und ist wichtig für die Regeneration von Haut, Muskeln, Nerven und DNA. Sie soll darüber hinaus die Durchblutung der Kopfhaut verbessern und wird bei durchschnittlichem Energiebedarf eines erwachsenen Körpers in einer Menge von 6.6 mg benötigt, um eine Energiemenge von 1'000 Kilokalorien für seine Zellen, Gewebe und Organe zu erzeugen. Damit beträgt der Bedarf für Frauen 13 bis 15 mg pro Tag und für Männer 15 bis 20 mg pro Tag.

Eine weitere Ausgestaltungsvariante eines erfindungsgemäßen Haarwuchspräparates umfasst nebst den bisherigen Grundsubstanzen oder auch optionalen Substanzen auch Folsäure, die auch unter dem Trivialnamen Vitamin B₉ bekannt und ein weiteres, lichtempfindliches Vitamin aus dem B-Komplex ist. Die Folsäure ist an der Synthese von DNS-Bausteinen beteiligt und spielt bei der Zellteilung eine entscheidende Rolle. Sie ist für den menschlichen Organismus essenziell und kann nicht von ihm selbst hergestellt werden. Folsäure-Mangel kann unter anderem zu Anämie führen und begünstigt in der Embryonalentwicklung die Entstehung von Neuralrohrdefekten wie eine Spina bifida oder Anenzephalie. Die empfohlene Tagesdosis nach RDA (Recommended Daily Allowance) oder aber auch nach den D-A-CH-Referenzwerten für die Nährstoffzufuhr beträgt 200 µg. In einigen Staaten, wie z.B. auch der Schweiz, ist eine Beimengungspflicht gesetzlich vorgesehen.

Eine weiterhin bevorzugte Zusammensetzungsvariante eines erfindungsgemäßen Haarwuchspräparates umfasst neben den Grundsubstanzen oder einer beliebigen Kombination der Grundsubstanzen mit den optionalen Substanzen auch das Coenzym Q10, auch bekannt unter der Bezeichnung Ubichinon-10. Es ist ein Chinon-Derivat, strukturell verwandt mit Vitamin K und Vitamin E und eine körpereigene Substanz, die teilweise über die Nahrung aufgenommen wird, aber auch im Körper selbst produziert wird. Q10 ist essenziell bei der Umwandlung in jeder menschlichen Zelle der Energie aus der Nahrung in körpereigene Energie (ATP). Q10 ist ein wesentlicher Cofaktor der Atmungskette in den Mitochondrien der Zelle und dient des Weiteren als wichtigstes lipophiles Antioxidans bei Stress und degenerativen Prozessen als Radikalfänger. Ein permanenter Q10-Mangel kommt selten vor, um jedoch einen Mangel zu vermeiden oder auszugleichen, ist von den meisten Wissenschaftlern eine Tagesdosis in Höhe von 30-200 mg pro Tag empfohlen.

Eine weiterhin bevorzugte Zusammensetzungsvariante eines erfindungsgemäßen Haarwuchspräparates umfasst neben den Grundsubstanzen oder einer beliebigen Kombination der Grundsubstanzen mit den optionalen Substanzen auch Omega-3-Fettsäuren. Diese sind essenzielle Stoffe für die menschliche Ernährung und können vom Körper nicht selbst hergestellt werden. Es werden ihnen u.a. eine Senkung kardiovaskulärer Risiken, Krebsvorbeugung, Entzündungshemmung, Vorbeugung von altersbedingten degenerativen Erkrankungen und Demenz nachgesagt. Insbesondere von den Omega-3-Fettsäuren Docosahexaensäure (DHA) und Eicosapentaensäure (EPA) empfiehlt die Europäische Behörde für Lebensmittelsicherheit (EFSA) und die Deutsche Gesellschaft für Ernährung (DGE) eine nahrungsbezogene tägliche Aufnahme von 250 mg. Die Beimengung der Omega-3-Fettsäuren erfolgt vorzugsweise mittels Mikrokapseln, welche einer vorzugsweise pulvrigen Zusammensetzung bzw. Konsistenz eines erfindungsgemäßen Haarwuchspräparates beigemengt werden und welche die Omega-3-Fettsäuren beinhalten.

Eine bevorzugte Zusammensetzung eines erfindungsgemäßen Haarwuchspräparates umfasst des Weiteren Guarkernmehl, an welches die offenbarten Substanzen und Substanzenzusammensetzungen gebunden werden. Guarkernmehl wird aus den Samen der Guarbohne hergestellt und besteht überwiegend aus langkettigen Kohlenhydraten, die auf charakteristische Weise aus den Einfachzuckern Mannose und Galaktose zusammengesetzt sind. Die langkettigen Verbindungen können sehr große Wassermengen binden. Dieses und die schleimige Konsistenz sind vermutlich die Ursache für die Beobachtung, dass die Substanzen und Substanzenzusammensetzungen eines erfindungsgemäßen Haarwuchspräparates deutlich besser bis in den Dünndarm transportiert werden. Im Unterschied zum Magen oder Zwölffingerdarm findet im Dünndarm eine auf fast 100% erhöhte Aufnahme der Substanzen und Substanzenzusammensetzungen statt, ohne unerwünschte Interaktionen. Darüber hinaus bringt das Guarkernmehl den positiven Nebeneffekt mit sich, dass es darmpflegend wirkt. Das Guarkernmehl erfüllt auf diese Weise erfindungsgemäß die Funktion eines Retarders, der die Resorption der Nährstoffe dort stattfinden lässt, wo sie am effektivsten ist.

Bei allfällig auftretenden allergischen Reaktionen auf das Guarkernmehl wird bei der Zusammensetzung eines oben beschriebenen bevorzugten erfindungsgemäßen Haarwuchspräparates vorzugsweise auf Johannisbrotkernmehl ausgewichen.

Aus all diesen Erkenntnissen und erfinderischen Überlegungen heraus ergibt sich eine optionale und beispielhafte Komplettvariante eines erfindungsgemäßen Haarwuchspräparates, beispielsweise für einen erwachsenen Menschen mit folgenden beispielhaften, nicht zwingend kumulativen Substanzen oder Substanzenzusammensetzungen und mit folgenden ebenfalls beispielhaften bevorzugten Tagesdosen und Tagesdosen-Bereichen:

| **Nährstoff** | | **Bevorzugte Tagesdosis** | **Bereich Tagesdosis** |
|---|---|---|---|
| **Vitamine** | | | |
| Biotin | | 5.0 mg | 0.1-40.0 mg |
| Folsäure | | 200.0 µg | 100.0-2'000.0 µg |
| Natürliche Carotinoide | | 8.0 mg | 1.0-160.15 mg |
| | davon Alpha-Carotin | 70.0 µg | 1.0-100'000.0 µg |
| | davon Cryptoxanthin | 15.0 µg | 1.0-50.0 µg |
| | davon Lutein | 6.0 mg | 1.0-15.0 mg |
| | davon Beta-Carotin | 1.9 mg | 1.0-45.0 mg |
| | davon Zeaxanthin | 15.0 µg | 1. 0-100.0 µg |
| Natürliches Vitamin E | | 300.0 mg | 1.0-1'000.0 mg |
| | davon Alpha-Tocopherol | 262.0 mg | 85-95% |
| | davon Gamma-Tocopherol | 30.0 mg | 5-15% |
| Niacin | | 50.0 mg | 1.0-500.0 mg |
| Pantothensäure | | 10.0 mg | 1.0-1'000.0 mg |
| Vitamin A (Retinoide) | | 1.0 mg | 1'000-40'000 IE |
| Vitamin B₁ (Thiamin) | | 5.0 mg | 1.0-200.0 mg |
| Vitamin B₂ (Riboflavin) | | 5.0 mg | 1.0-100.0 mg |
| Vitamin B₆ | | 10.0 mg | 1.0-480.0 mg |
| Vitamin B₁₂ | | 35.0 µg | 1.0-1'000.0 µg |
| Vitamin C | | 1'500.0 mg | 1.0-10'000.0 mg |
| Vitamin D3 | | 7 µg | 1.0-100.0 µg |

| **Spurenelemente** | | | |
|---|---|---|---|
| Chrom | | 50.0 µg | 1.0-300.0 µg |
| Eisen | | 2.5 mg | 1.0-100.0 mg |
| Jod | | 50.0 µg | 1.0-1'000.0 µg |
| Kupfer | | 2.0 mg | 0.1-5.0 mg |
| Mangan | | 5.0 mg | 1.0-50.0 mg |
| Molybdän | | 50.0 µg | 1.0-1'000.0 µg |
| Selen | | 200.0 µg | 1.0-400.0 µg |
| Vanadium | | 50.0 µg | 1.0-200.0 µg |
| Zink | | 25.0 mg | 1.0-100.0 mg |

| **Mineralstoffe** | | | |
|---|---|---|---|
| Calcium | | 800.0 mg | 1.0-1'500.0 mg |
| Kalium | | 220.0 mg | 1.0-5'000.0 mg |
| Magnesium | | 400.0 mg | 1.0-1'500.0 mg |
| Silicium | | 26.0 mg | 1.0-50.0 mg |

| **Quasi-Vitamine** | | | |
|---|---|---|---|
| a-Liponsäure | | 20.0 mg | 1.0-1'000.0 mg |
| Cholin | | 40.0 mg | 1.0-10'000.0 mg |
| Inositol | | 30.0 mg | 1.0-3'000.0 mg |
| L-Carnitin | | 250.0 mg | 1.0-3'000.0 mg |
| p-Aminobenzoesäure (PABA) | | 10.0 mg | 1.0-4'000.0 mg |
| Coenzym Q10 (Ubichinon-10) | | 100.0 mg | 1.0-600.0 mg |
| Omega-3-Fettsäuren | | 250.0 mg | 100.0-600.0 mg |

| **Aminosäuren** | | | |
|---|---|---|---|
| L-Cystein | | 1'000.0 mg | 1.0-2'000.0 mg |
| L-Methionin | | 400.0 mg | 1.0-5'000.0 mg |
| Gamma-Aminobuttersäure (GABS) | | 1.0 mg | 0.1-5'000.0 mg |

| **Pflanzenextrakte** | | | |
|---|---|---|---|
| Sylimarin | | 150.0 mg | 1.0-220.0 mg |
| Zitrusbioflavonoide | | 200.0 mg | 1.0-400.0 mg |

| **Ballaststoffe** | | | |
|---|---|---|---|
| Guar | | 200.0 mg | 1.0-500.0 mg |

Als Tests und Messungen kommen beispielsweise die folgenden in Betracht: Blut-, Urin-, Creatinin-, Speichel-, Haar-, Stuhl- und Atemtests, der Letztere insbesondere zur Ermittlung des Säuregehaltes bzw. des PH-Wertes in der ausgestoßenen Atemluft. Den im Folgenden beschriebenen Ausgestaltungsvarianten eines erfindungsgemäßen Haarwuchspräparates liegt unter anderem die Erkenntnis zugrunde, dass eine Übersäuerung der menschlichen Organismen weitverbreitet ist und so der Aufnahme mancher Vitamine, Spurenelemente, Mineralstoffe und Aminosäuren im Wege steht.

Der oben erwähnte Bluttest setzt sich vorzugsweise aus folgenden Einzeltests zusammen: Blutbild, Blutsenkung, Schilddrüsen- und Nierenfunktionsparameter, Eisen im Serum, Eisenbindungskapazität, Zink und Selen aus dem Vollblut, Calcium aus dem Serum und dem Vollblut, Transaminasen und Immunglobulin E (IgE)-Spiegel.

Als Fragebogen oder auch nur als Anleitung für die Erfassung der Kundendaten kommt beispielsweise in Betracht, das Geschlecht, die Körpergröße, das Gewicht, das Alter, den Gesundheitszustand, die Einnahme von Medikamenten sowie sonstige Ess-, Trink- und Lebensgewohnheiten abzufragen. Des Weiteren wird beispielsweise auch abgefragt, was sich der Kunde betreffend seines Haarwuchses wünscht.

Es ist erfindungsgemäß vorgesehen, in Abhängigkeit der Tests und Messungen, aber optional auch bereits in Abhängigkeit nur der Antworten zu dem Fragebogen, die Grunddosierung des erfindungsgemäßen Haarwuchspräparates anzupassen. So ist es beispielsweise vorgesehen, einen Mann auf eine Tagesdosis von 25.0 mg oder gar 30.0 mg Zink zu setzen, im Unterschied zu einer Frau, bei der 20.0 mg Zink vorgesehen werden. Das Zink wird bei den Männern erhöht, da sie durch die Spermaproduktion einen gegenüber Frauen erhöhten Bedarf haben.

Des Weiteren kann es vorgesehen sein, für eine Frau im Unterschied zu einem Mann eine Tagesdosis von 3.0 mg Gamma-Aminobuttersäure statt nur 1.0 mg vorzusehen. Die Gamma-Aminobuttersäure GABS wird bei Frauen erhöht, da sie unter dem prämenstruellen Syndrom leiden können und dieses durch die Gabe von GABS gelindert wird. Zusätzlich dazu haben Frauen im Unterschied zum Mann eine geringere Proteinreserve. Deshalb brauchen sie mehr GABS. Die GABS hat in Kombination mit Vitamin B₆ und L-Cystein eine positive, stärkende Wirkung. Der Mann hingegen hat häufig eine Glatze oder Teilglatze und hat dadurch einen erhöhten Bedarf an L-Cystein, weshalb er wiederum weniger GABS benötigt. Bei demjenigen, welcher mehr als drei Mal pro Woche Alkohol konsumiert, wird erhöht GABS verabreicht, da GABS das Verlangen nach Alkohol durch seine nervenberuhigende Wirkung reduzieren kann.

Weiterhin, ebenfalls das unterschiedliche Geschlecht betreffend, kann es erfindungsgemäß vorgesehen sein, für einen Mann nur 1.0 mg Eisen statt zum Beispiel 2.5 mg für eine Frau vorzusehen. Das Eisen kann beim Mann etwas reduziert werden, da er keinen Eisenverlust über die Menstruation hat und von seinen durchschnittlichen Ernährungsgewohnheiten meistens mehr Fleisch als eine Frau verzehrt.

Die Körpergröße und das Gewicht betreffend, ist es weiterhin erfindungsgemäß vorgesehen, 13.0 mg L-Methionin pro kg Körpergewicht der Zusammensetzung beizugeben.

Je nach Alter der Kundin oder des Kunden, wird eine altersabgestufte Menge von Vitamin B₁₂ vorgesehen, die sich grundsätzlich in einem Bereich von 1.0-1'000.0 µg bewegen kann, vorzugsweise jedoch in einem Bereich von 20.0 µg bis 50.0 µg bewegt und vorzugsweise 35.0 µg beträgt. Weiterhin vorzugsweise sind bei einem Alter von 18-35 Jahren 20.0 µg, bei einem Alter von 35-60 Jahren eben 35.0 µg und bei einem Alter von 60-99 Jahren 50.0 µg vorgesehen.

Bei einer vorhandenen Schilddrüsenunterfunktion kann die Menge an Jod auf 200.0 µg erhöht werden.

Bei einer bereits vorhandenen Krebserkrankung kann die bevorzugte und in der Tabelle angegebene Menge von 10.0 mg Vitamin B₆ auf 25.0 mg erhöht werden. Des Weiteren die Menge von 0.2 mg Folsäure auf 0.4 mg. Q10 kann von 100.0 mg auf 150.0 mg und GABS von 1.0 mg auf 2.0 mg. Zusätzlich dazu kann Vitamin B₁₂ von 35.0 µg auf 50.0 µg erhöht werden.

Bei Diabetes kann das L-Cystein von seiner bevorzugten und in der Tabelle angegebenen Menge von 1'000.0 mg auf 400.0 mg reduziert werden. Darüber hinaus können Vitamin B₁ von 5.0 mg auf 50.0 mg und Vitamin B₆ von 10.0 mg ebenfalls auf 50.0 mg erhöht werden. Des Weiteren Chrom von 50.0 µg auf 200.0 µg.

Bei Stress, psychischem oder körperlichem, kann das Vitamin C von 1'500.0 mg auf 2'000.0 mg erhöht werden und Folsäure von 200.0 µg auf 800.0 µg.

Wenn eine Kundin mehr als drei Mal pro Woche Sport treibt, so kann das Zink von der in der Tabelle als bevorzugt angegebenen Menge von 25.0 mg auf 30.0 mg erhöht werden. Wenn diese Sporttätigkeit bei einem Mann zutrifft, so kann auf 40.0 mg erhöht werden.

Wenn die Kundin oder der Kunde mehr als drei Mal pro Woche Alkohol trinkt, kann Zink von der bevorzugten und in der Tabelle angegebenen Menge von 25.0 mg auf 30.0 mg erhöht werden und GABS von 1.0 mg auf 3.0 mg. Ebenfalls kann die Dosis Niacin von 50.0 mg auf 300.0 mg erhöht werden.

Bei einer Kundin oder einem Kunden, die oder der regelmäßig raucht, kann durch den Zigarettenrauch ein Zinkmangel auftreten, der wiederum einen Kupferüberschuss oder gar eine Kupfervergiftung zur Folge haben kann. Demzufolge kann bei Rauchern das Kupfer von der bevorzugten und in der Tabelle ausgewiesenen Menge von 2.0 mg auf 1.0 mg reduziert werden. Des Weiteren kann bei Rauchern das Vitamin C von 1'500.0 mg auf 2'000.0 mg erhöht werden. Weiterhin Folsäure von 0.2 mg auf 0.4 mg, Vitamin B₆ von 10.0 mg auf 25.0 mg und Vitamin B₁₂ von 35.0 µg auf 40.0 µg. Des Weiteren kann auch die Selengabe von 200.0 µg auf 250.0 µg erhöht werden.

Ein erfindungsgemäßes Haarwuchspräparat kann optional von einer Entsäuerung des Darmtraktes bzw. des Organismus begleitet oder vorangegangen sein. Gleichzeitig kann Silicium, Zink und Eisen gegeben werden, die beiden letzteren Spurenelemente vorzugsweise in chelatierter Form.

Falls erforderlich, kann für eine optimale Aufnahme der Substanzen und Substanzenzusammensetzungen eine vorherige Darmsanierung vorgesehen sein.

Grundsätzlich jedoch wäre es aber auch möglich, ein erfindungsgemäßes Haarwuchspräparat auch intramuskulär oder subkutan oder intravenös zu verabreichen und vielleicht betreffend bestimmter Substanzen mittels Depotspritzen.

Ein erfindungsgemäßes Haarwuchspräparat wird vorzugsweise in einer allgemeingültigen Basis-Version mit Durchschnittsdosierungen für Frauen und einer allgemeingültigen Basis-Version mit Durchschnittsdosierungen für Männer her- bzw. bereitgestellt. Nach Auswertung der Tests und Messungen sowie der Fragebögen, kann diese Basis-Version mit den erforderlichen Differenzsubstanzen oder Differenzdosierungen zu dem erfindungsgemäß individuell maßgeschneiderten Haarwuchspräparat ergänzt werden.

Die offenbarten unterschiedlichen Ausgestaltungsvarianten eines erfindungsgemäßen Haarwuchspräparates sind hinsichtlich der optionalen Substanzen beliebig miteinander kombinierbar.

Die vorliegende Anmeldung offenbart ein Verfahren zum kundenspezifischen Anpassen eines wie offenbarten Haarwuchspräparates, mit folgenden grundsätzlichen Verfahrensschritten:
a) - Bereitstellen einer allgemeingültigen Haarwuchspräparat-Version mit Durchschnittsdosierungen für Frauen;
b) - Bereitstellen einer allgemeingültigen Haarwuchspräparat-Version mit Durchschnittsdosierungen für Männer;
c) - Beantwortenlassen von Fragebögen durch eine Kundin oder einen Kunden;
d) - falls erforderlich, Durchführen von Tests und Messungen;
e) - Ermitteln anhand der Fragebögen-Auswertungen und der Tests und Messungen der Differenzsubstanzen und Differenzdosierungen;
f) - Auswählen der allgemeingültigen Haarwuchspräparat-Version für Frauen im Falle einer Kundin;
g) - Auswählen der allgemeingültigen Haarwuchspräparat-Version für Männer im Falle eines Kunden;
h) - Anpassen der ausgewählten Haarwuchspräparat-Version an die individuelle Kundin oder an den individuellen Kunden durch Beifügen der ermittelten Differenzsubstanzen und Differenzdosierungen.

Ein erfindungsgemäßes Haarwuchspräparat bringt folgende Vorteile, viele davon durch Probanden belegt:
- Das Haarwuchspräparat ist individuell auf die Kundin oder den Kunden abgestimmt.
- Es werden Vitalstoff-Mängel ermittelt.
- Es werden so gut wie alle erforderlichen Vitalstoffe abgedeckt, mit Spitzendosierungen bei den fehlenden.
- Es ist gut verträglich.
- Es steigert die Gesundheit und das Wohlbefinden.
- Probanden fühlen sich weniger müde und haben mehr Energie.
- Der Haarwuchs wird angeregt, sowohl die Anzahl der Haare betreffend, als auch deren Stärke und Gesundheit.
- Das Haar gewinnt an Glanz.
- Dünnes Haar wird voller.
- Brüchiges Haar wird geschmeidig.
- Schuppende Kopfhaut wird wieder gesund.
- Die Geschwindigkeit des Haarwuchses wird innerhalb einer Anagenphase beschleunigt.

Weitere oder vorteilhafte Ausgestaltungen eines erfindungsgemäßen Haarwuchspräparats bilden die Gegenstände der abhängigen Ansprüche.

Die Bezugszeichenliste ist Bestandteil der Offenbarung.

Anhand von einer Figur wird ein Teilaspekt der Erfindung symbolisch und beispielhaft näher erläutert. Sie stellt schematische und beispielhafte Darstellungen dar und ist nicht maßstabsgetreu, auch in der Relation der einzelnen Bestandteile zueinander nicht. Gleiche Bezugszeichen bedeuten den gleichen Gegenstand oder Komponente, Bezugszeichen mit unterschiedlichen Indizes geben funktionsgleiche oder ähnliche Gegenstände oder Komponenten an.

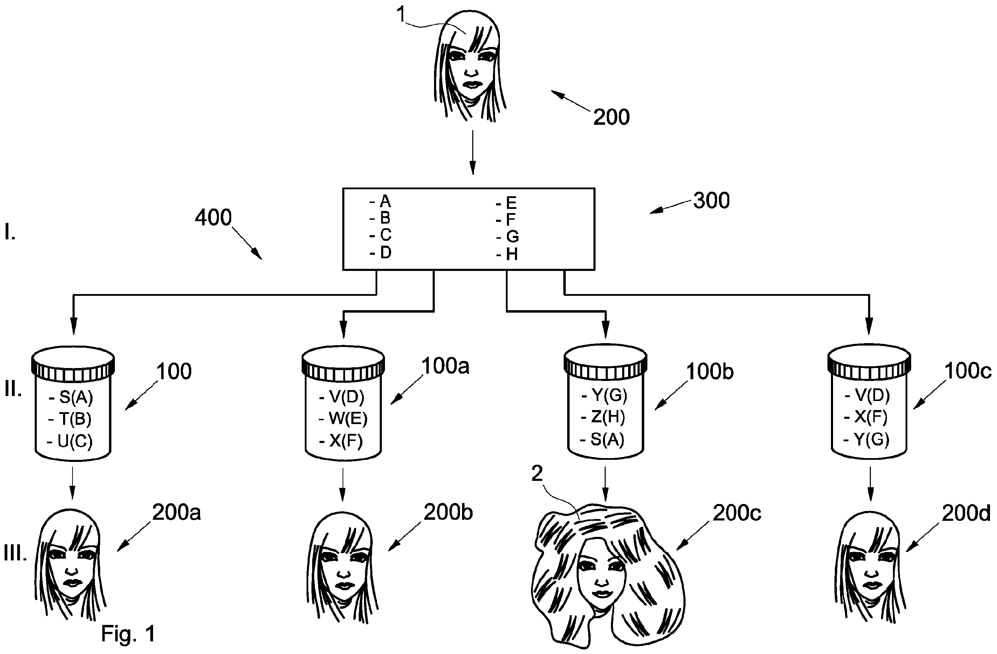

Es zeigt dabei
Fig. 1 eine schematische Darstellung einer beispielhaften Kundenanpassung eines erfindungsgemäßen Haarwuchspräparates.

In der Fig. 1 ist exemplarisch eine Kundin 200 dargestellt, die wegen eines schütteren Haarwuchses 1 Hilfe sucht. Eine erste Phase I ist eine Kundenerfassung oder Bedarfsermittlung 300, die Tests, Messungen und Fragebögen-Auswertungen A-H umfasst.

Eine zweite Phase II einer Präparat-Anpassung 400 stellt dar, dass ein erstes Haarwuchspräparat 100 Substanzen oder Substanzenzusammensetzungen S-U umfasst, die in Funktion der Tests A-C ausgewählt wurden. Beispielhaft ist ein weiteres, zweites Haarwuchspräparat 100a dargestellt, bei dem Substanzen oder Substanzenzusammensetzungen V-X in Funktion der Tests D-F ausgewählt wurden. Weiterhin beispielhaft ist ein drittes Haarwuchspräparat 100b dargestellt, bei dem eine Substanz oder Substanzenzusammensetzung Y in Funktion des Tests G ausgewählt ist, sowie eine Substanz oder Substanzenzusammensetzung Z in Funktion des Tests H und die Substanz oder Substanzenzusammensetzung S in Funktion des Tests A. Eine vierte und letzte beispielhafte Ausgestaltungsvariante eines Haarwuchspräparates 100c umfasst die Substanz oder Substanzenzusammensetzung V in Funktion des Tests D, die Substanz oder Substanzenzusammensetzung X in Funktion des Tests F und die Substanz oder Substanzenzusammensetzung Y in Funktion des Tests G.

Eine dritte Phase III zeigt symbolisch und beispielhaft, dass die Kundin 200 aus der Phase I durch die Einnahme des Haarwuchspräparates 100 zwar zu einer "veränderten" Kundin 200a geworden ist, jedoch hinsichtlich eines verbesserten Haarwuchses noch kein großer Erfolg eingetreten ist. Das Gleiche gilt entsprechend für die Haarwuchspräparate 100a und 100c beziehungsweise für Kundinnen 200b und 200d. Bei dem Haarwuchspräparat 100b hingegen ist aus der Kundin 200 eine Kundin 200c mit einem kräftigen und voluminösen Haarwuchs 2 geworden. Eine Zeitachse der Phasen I-III beträgt in jedem Fall mehrere Monate.

### Bezugszeichenliste

1 - schütterer Haarwuchs
2 - kräftiger, voluminöser Haarwuchs
100, 100a-100c - Haarwuchspräparat
200, 200a-200d - Kundin
300 - Kundenerfassung, Bedarfsermittlung
400 - Präparat-Anpassung
I - erste Phase
II - zweite Phase
III - dritte Phase
A-H - Tests, Messungen, Fragebögen-Auswertungen
S-Z - Substanz oder Substanzenzusammensetzung

## Patentansprüche

1. Haarwuchspräparat zur oralen Einnahme als Tagesdosis, L-Cystein und Zink umfassend, **dadurch gekennzeichnet, dass** das Haarwuchspräparat 1'000.0 mg bis 2'000.0 mg L-Cystein und Kupfer, Calcium, Magnesium, Silicium, Eisen und Cholecalciferol umfasst, sowie Guarkern- oder Johannisbrotkernmehl zur retardierten Resorption im Dünndarm **und dass** eine erste Basis-Version des Haarwuchspräparats für Frauen mit 20.0 mg Zink und 2.5 mg Eisen oder eine zweite Basis-Version des Haarwuchspräparats für Männer mit 25.0 mg Zink und 1.0 mg Eisen auswählbar sind **und dass** die zusammensetzenden Substanzen oder Substanzenzusammensetzungen der ausgewählten Basis-Version aufgrund von Fragebögen-Auswertungen und Blut-, Urin-, Creatinin-, Speichel-, Haar-, Stuhl- und Atemtests an die ermittelten individuellen organischen Nährstoff-Versorgungs-Voraussetzungen einer Kundin oder eines Kunden adaptiert bzw. ergänzt sind.

2. Haarwuchspräparat nach Anspruch 1, **dadurch gekennzeichnet, dass** das Haarwuchspräparat natürliches Vitamin E in Form von Alpha-Tocopherol und Gamma-Tocopherol umfasst.

3. Haarwuchspräparat nach einem der vorhergehenden Ansprüche, **dadurch gekennzeichnet, dass** das Haarwuchspräparat Ascorbinsäure umfasst.

4. Haarwuchspräparat nach einem der vorhergehenden Ansprüche, **dadurch gekennzeichnet, dass** das Haarwuchspräparat Biotin umfasst.

5. Haarwuchspräparat nach einem der vorhergehenden Ansprüche, **dadurch gekennzeichnet, dass** das Haarwuchspräparat L-Methionin umfasst.

6. Haarwuchspräparat nach einem der vorhergehenden Ansprüche, **dadurch gekennzeichnet, dass** das Haarwuchspräparat Pantothensäure umfasst.

7. Haarwuchspräparat nach einem der vorhergehenden Ansprüche, **dadurch gekennzeichnet, dass** das Haarwuchspräparat Pyridoxin und/oder Pyridoxal und/oder Pyridoxamin umfasst.

8. Haarwuchspräparat nach einem der vorhergehenden Ansprüche, **dadurch gekennzeichnet, dass** das Haarwuchspräparat Cobalamin umfasst.

9. Haarwuchspräparat nach einem der vorhergehenden Ansprüche, **dadurch gekennzeichnet, dass** das Haarwuchspräparat Selen umfasst.

10. Haarwuchspräparat nach einem der vorhergehenden Ansprüche, **dadurch gekennzeichnet, dass** das Haarwuchspräparat Nicotinsäure umfasst.

11. Haarwuchspräparat nach einem der vorhergehenden Ansprüche, **dadurch gekennzeichnet, dass** das Haarwuchspräparat Folsäure umfasst.

12. Haarwuchspräparat nach einem der vorhergehenden Ansprüche, **dadurch gekennzeichnet, dass** das Haarwuchspräparat Ubichinon-10 umfasst.

13. Haarwuchspräparat nach einem der vorhergehenden Ansprüche, **dadurch gekennzeichnet, dass** das Haarwuchspräparat Omega-3-Fettsäuren umfasst, die einer pulvrigen Zusammensetzung des Haarwuchspräparats in Form von Mikrokapseln beigemengt sind, welche die Omega-3-Fettsäuren beinhalten.

14. Haarwuchspräparat nach einem der vorhergehenden Ansprüche, **dadurch gekennzeichnet, dass** das Haarwuchspräparat natürliche Carotinoide, Retinoide, Thiamin, Riboflavin, Chrom, Jod, Mangan, Molybdän, Vanadium, Calcium, Kalium, a-Liponsäure, Cholin, Inositol, L-Carnitin, p-Aminobenzoesäure, Gamma-Aminobuttersäure, Sylimarin und Zitrusbioflavonoide umfasst.

15. Verfahren zur kundenspezifischen Anpassung eines Haarwuchspräparats mindestens mit einer adaptierbaren Substanzenzusammensetzung nach Anspruch 1 oder nach einem der vorhergehenden Ansprüche 2-14, **dadurch gekennzeichnet, dass** folgende Verfahrensschritte ausgeführt werden:
a) - Bereitstellen einer Basis-Version des Haarwuchspräparats für Frauen mit 20.0 mg Zink und 2.5 mg Eisen;
b) - Bereitstellen einer Basis-Version des Haarwuchspräparats für Männer mit 25.0 mg Zink und 1.0 mg Eisen;
c) - Beantwortenlassen von Fragebögen durch eine Kundin oder einen Kunden;
d) - Durchführen von Blut-, Urin-, Creatinin-, Speichel-, Haar-, Stuhl- und Atemtests;
e) - Ermitteln anhand der Fragebögen-Auswertungen und der Blut-, Urin-, Creatinin-, Speichel-, Haar-, Stuhl- und Atemtests der Differenzsubstanzen und Differenzdosierungen zu den Dosierungen der Basis-Version;
f) - Auswählen der Basis-Version des Haarwuchspräparats für Frauen im Falle einer Kundin;
g) - Auswählen der Basis-Version des Haarwuchspräparats für Männer im Falle eines Kunden;
h) - Anpassen der ausgewählten Basis-Version des Haarwuchspräparats an die ermittelten individuellen organischen Nährstoff-Versorgungs-Voraussetzungen einer Kundin oder eines Kunden, durch Beifügen der ermittelten Differenzsubstanzen und Differenzdosierungen zu den Dosierungen der ausgewählten Basis-Version.

16. Stoffgemisch enthaltend die Stoffe L-Cystein und Zink, zur Anwendung bei der Therapie gegen Haarausfall und für Haarwuchs-Anregung, **dadurch gekennzeichnet, dass** das Stoffgemisch 1'000.0 mg bis 2'000.0 mg L-Cystein enthält **und dass** das Stoffgemisch noch Kupfer, Calcium, Magnesium, Silicium, Eisen und Cholecalciferol enthält, sowie Guarkern- oder Johannisbrotkernmehl zur retardierten Resorption im Dünndarm **und dass** eine Basis-Version des Stoffgemisches für Frauen mit 20.0 mg Zink und 2.5 mg Eisen oder eine Basis-Version des Stoffgemisches für Männer mit 25.0 mg Zink und 1.0 mg Eisen auswählbar sind **und dass** die zusammensetzenden Substanzen oder Substanzenzusammensetzungen aufgrund von Fragebögen-Auswertungen und Blut-, Urin-, Creatinin-, Speichel-, Haar-, Stuhl- und Atemtests an die ermittelten individuellen organischen Nährstoff-Versorgungs-Voraussetzungen einer Kundin oder eines Kunden adaptiert bzw. ergänzt sind.

17. Stoffgemisch zur Verwendung entsprechend Anspruch 16, **dadurch gekennzeichnet, dass** das Stoffgemisch natürliches Vitamin E in Form von Alpha-Tocopherol und Gamma-Tocopherol enthält.

18. Stoffgemisch zur Verwendung entsprechend Anspruch 16 oder 17, **dadurch gekennzeichnet, dass** das Stoffgemisch Ascorbinsäure enthält.

19. Stoffgemisch zur Verwendung entsprechend einem der vorhergehenden Ansprüche 17-18, **dadurch gekennzeichnet, dass** das Stoffgemisch Biotin enthält.

20. Stoffgemisch zur Verwendung entsprechend einem der vorhergehenden Ansprüche 17-19, **dadurch gekennzeichnet, dass** das Stoffgemisch L-Methionin enthält.

21. Stoffgemisch zur Verwendung entsprechend einem der vorhergehenden Ansprüche 17-20, **dadurch gekennzeichnet, dass** das Stoffgemisch Pantothensäure enthält.

22. Stoffgemisch zur Verwendung entsprechend einem der vorhergehenden Ansprüche 17-21, **dadurch gekennzeichnet, dass** das Stoffgemisch Pyridoxin und/oder Pyridoxal und/oder Pyridoxamin enthält.

23. Stoffgemisch zur Verwendung entsprechend einem der vorhergehenden Ansprüche 17-22, **dadurch gekennzeichnet, dass** das Stoffgemisch Cobalamin enthält.

24. Stoffgemisch zur Verwendung entsprechend einem der vorhergehenden Ansprüche 17-23, **dadurch gekennzeichnet, dass** das Stoffgemisch Selen enthält.

25. Stoffgemisch zur Verwendung entsprechend einem der vorhergehenden Ansprüche 17-24, **dadurch gekennzeichnet, dass** das Stoffgemisch Nicotinsäure enthält.

26. Stoffgemisch zur Verwendung entsprechend einem der vorhergehenden Ansprüche 17-25, **dadurch gekennzeichnet, dass** das Stoffgemisch Folsäure enthält.

27. Stoffgemisch zur Verwendung entsprechend einem der vorhergehenden Ansprüche 17-26, **dadurch gekennzeichnet, dass** das Stoffgemisch Ubichinon-10 enthält.

28. Stoffgemisch zur Verwendung entsprechend einem der vorhergehenden Ansprüche 17-27, **dadurch gekennzeichnet, dass** das Stoffgemisch Omega-3-Fettsäuren enthält, die einer pulvrigen Zusammensetzung des Stoffgemischs in Form von Mikrokapseln beigemengt sind, welche die Omega-3-Fettsäuren beinhalten.

29. Stoffgemisch zur Verwendung entsprechend einem der vorhergehenden Ansprüche 17-28, **dadurch gekennzeichnet, dass** das Stoffgemisch natürliche Carotinoide, Retinoide, Thiamin, Riboflavin, Chrom, Jod, Mangan, Molybdän, Vanadium, Calcium, Kalium, a-Liponsäure, Cholin, Inositol, L-Carnitin, p-Aminobenzoesäure, Gamma-Aminobuttersäure, Sylimarin und Zitrusbioflavonoide enthält.

## Claims

1. Hair growth preparation for oral administration as daily dose, comprising L-cysteine and zinc, **characterized in that** the hair growth preparation comprises 1'000.0 mg to 2'000.0 mg L-cysteine and copper, calcium, magnesium, silicium, iron and cholecalciferol, as well as guar gum or locust bean gum for the retarded resorption in the small intestine **and that** a first basic version of the hair growth preparation for women, with 20.0 mg zinc and 2.5 mg iron or a second basic version of the hair growth preparation for men, with 25.0 mg zinc and 1.0 mg iron are selectable **and that** the composing substances or compositions of substances of the selected basic version are adapted or supplemented to the determined individual organic nutrient supply requirements of a female customer or male customer, on basis of evaluations of questionnaires and blood, urine, creatinine, saliva, hair, stool and breath tests.

2. Hair growth preparation according to claim 1, **characterized in that** the hair growth preparation comprises natural vitamin E in the form of alpha-tocopherol and gamma-tocopherol.

3. Hair growth preparation according to one of the preceding claims, **characterized in that** the hair growth preparation comprises ascorbic acid.

4. Hair growth preparation according to one of the preceding claims, **characterized in that** the hair growth preparation comprises biotin.

5. Hair growth preparation according to one of the preceding claims, **characterized in that** the hair growth preparation comprises L-methionine.

6. Hair growth preparation according to one of the preceding claims, **characterized in that** the hair growth preparation comprises pantothenic acid.

7. Hair growth preparation according to one of the preceding claims, **characterized in that** the hair growth preparation comprises pyridoxine and/or pyridoxal and/or pyridoxamine.

8. Hair growth preparation according to one of the preceding claims, **characterized in that** the hair growth preparation comprises cobalamin.

9. Hair growth preparation according to one of the preceding claims, **characterized in that** the hair growth preparation comprises selenium.

10. Hair growth preparation according to one of the preceding claims, **characterized in that** the hair growth preparation comprises nicotinic acid.

11. Hair growth preparation according to one of the preceding claims, **characterized in that** the hair growth preparation comprises folic acid.

12. Hair growth preparation according to one of the preceding claims, **characterized in that** the hair growth preparation comprises ubiquinone-10.

13. Hair growth preparation according to one of the preceding claims, **characterized in that** the hair growth preparation comprises omega-3 fatty acids contained in micro capsules which are admixed to a powdery composition of the hair growth preparation.

14. Hair growth preparation according to one of the preceding claims, **characterized in that** the hair growth preparation comprises natural carotenoids, retinoids, thiamin, riboflavin, chromium, iodine, manganese, molybdenum, vanadium, calcium, potassium, a-lipoic acid, choline, inositol, L-carnitine, p-aminobenzoic acid, gamma aminobutyric acid, Sylimarin and citric bioflavonoids.

15. Procedure for the customized adaptation of a hair growth preparation with at least one adaptable substance composition according to claim 1 or according to one of the preceding claims 2-14, **characterized in that** the following procedure steps are carried out:
a) - providing of a basic version of the hair growth preparation for women, with 20.0 mg zinc and 2.5 mg iron;
b) - providing of a basic version of the hair growth preparation for men, with 25.0 mg zinc and 1.0 mg iron;
c) - letting respond to questionnaires by a female customer or male customer;
d) - executing of blood, urine, creatinine, saliva, hair, stool and breath tests;
e) - determining on basis of the questionnaire evaluations and the blood, urine, creatinine, saliva, hair, stool and breath tests of the difference substances and difference dosages to the dosages of the basic version;
f) - selecting the basic version of the hair growth preparation for women in the case of a female customer;
g) - selecting of the basic version of the hair growth preparation for men in the case of a male customer;
h) - adapting of the selected basic version of the hair growth preparation to the determined individual organic nutrient supply requirements of a female customer or male customer, by adding of the determined difference substances and difference dosages to the dosages of the selected basic version.

16. Mixture of substances containing the substances L-cysteine and zinc, for the application at the therapy against hair loss and for hair growth stimulation, **characterized in that** the mixture of substances contains 1'000.0 mg to 2'000.0 mg L-cysteine **and that** the mixture of substances contains also copper, calcium, magnesium, silicium, iron and cholecalciferol, as well as guar gum or locust bean gum for the retarded resorption in the small intestine **and that** a basic version of the mixture of substances for women, with 20.0 mg zinc and 2.5 mg iron or a basic version of the mixture of substances for men, with 25.0 mg zinc and 1.0 mg iron are selectable **and that** the composing substances or compositions of substances of the selected basic version are adapted or supplemented to the determined individual organic nutrient supply requirements of a female customer or male customer, on basis of evaluations of questionnaires and blood, urine, creatinine, saliva, hair, stool and breath tests.

17. Mixture of substances for the application according to claim 16, **characterized in that** the mixture of substances contains natural vitamin E in the form of alpha-tocopherol and gamma-tocopherol.

18. Mixture of substances for the application according to claim 16 or 17, **characterized in that** the mixture of substances contains ascorbic acid.

19. Mixture of substances according to one of the preceding claims 17-18, **characterized in that** the mixture of substances contains biotin.

20. Mixture of substances according to one of the preceding claims 17-19, **characterized in that** the mixture of substances contains L-methionine.

21. Mixture of substances according to one of the preceding claims 17-20, **characterized in that** the mixture of substances contains pantothenic acid.

22. Mixture of substances according to one of the preceding claims 17-21, **characterized in that** the mixture of substances contains pyridoxine and/or pyridoxal and/or pyridoxamine.

23. Mixture of substances according to one of the preceding claims 17-22, **characterized in that** the mixture of substances contains cobalamin.

24. Mixture of substances according to one of the preceding claims 17-23, **characterized in that** the mixture of substances contains selenium.

25. Mixture of substances according to one of the preceding claims 17-24, **characterized in that** the mixture of substances contains nicotinic acid.

26. Mixture of substances according to one of the preceding claims 17-25, **characterized in that** the mixture of substances contains folic acid.

27. Mixture of substances according to one of the preceding claims 17-26, **characterized in that** the mixture of substances contains ubiquinone-10.

28. Mixture of substances according to one of the preceding claims 17-27, **characterized in that** the mixture of substances contains omega-3 fatty acids contained in micro capsules which are admixed to a powdery composition of the mixture of substances.

29. Mixture of substances according to one of the preceding claims 17-28, **characterized in that** the mixture of substances contains natural carotenoids, retinoids, thiamin, riboflavin, chromium, iodine, manganese, molybdenum, vanadium, calcium, potassium, a-lipoic acid, choline, inositol, L-carnitine, p-aminobenzoic acid, gamma aminobutyric acid, Sylimarin and citric bioflavonoids.

## Revendications

1. Préparation pour la repousse des cheveux pour prise orale en dose quotidienne, comprenant de la L-cystéine et du zinc, **caractérisé en ce que** la préparation pour la repousse des cheveux comprend 1'000,0 mg à 2'000,0 mg de L-cystéine et cuivre, calcium, magnésium, silicium, fer et cholécalciférol, ainsi que de la gomme de guar ou de la gomme de caroube pour l'absorption retardée dans l'intestin grêle **et qu**'un premier basique version de la préparation pour la repousse des cheveux pour femmes avec 20,0 mg de zinc et 2,5 mg de fer ou une seconde version de base de la préparation pour la repousse des cheveux pour hommes avec 25,0 mg de zinc et 1,0 mg de fer sont sélectionnables **et que** les substances constitutives ou compositions de substances de la version de base sélectionnée sont adaptées ou sont complétées sur la base d'évaluations par questionnaire et de tests de sang, d'urine, de créatinine, de salive, de cheveux, de selles et d'haleine apport individuel déterminé de nutriments organiques d'une cliente ou d'un client.

2. Préparation pour la repousse des cheveux selon la revendication 1, **caractérisée en ce que** la préparation pour la repousse des cheveux comprend de la vitamine E naturelle sous forme d'alpha-tocophérol et de gamma-tocophérol.

3. Préparation pour la repousse des cheveux selon l'une des revendications précédentes, **caractérisée en ce que** la préparation pour la repousse des cheveux comprend de l'acide ascorbique.

4. Préparation pour la repousse des cheveux selon l'une des revendications précédentes, **caractérisée en ce que** la préparation pour la repousse des cheveux comprend de la biotine.

5. Préparation pour la repousse des cheveux selon l'une des revendications précédentes, **caractérisée en ce que** la préparation pour la repousse des cheveux comprend de la L-méthionine.

6. Préparation pour la repousse des cheveux selon l'une des revendications précédentes, **caractérisée en ce que** la préparation pour la repousse des cheveux comprend de l'acide pantothénique.

7. Préparation pour la repousse des cheveux selon l'une des revendications précédentes, **caractérisée en ce que** la préparation pour la repousse des cheveux comprend de la pyridoxine et/ou du pyridoxal et/ou de la pyridoxamine.

8. Préparation pour la repousse des cheveux selon l'une des revendications précédentes, **caractérisée en ce que** la préparation pour la repousse des cheveux comprend de la cobalamine.

9. Préparation pour la repousse des cheveux selon l'une des revendications précédentes, **caractérisée en ce que** la préparation pour la repousse des cheveux comprend du sélénium.

10. Préparation pour la repousse des cheveux selon l'une des revendications précédentes, **caractérisée en ce que** la préparation pour la repousse des cheveux comprend de l'acide nicotinique.

11. Préparation pour la repousse des cheveux selon l'une des revendications précédentes, **caractérisée en ce que** la préparation pour la repousse des cheveux comprend de l'acide folique.

12. Préparation pour la repousse des cheveux selon l'une des revendications précédentes, **caractérisée en ce que** la préparation pour la repousse des cheveux comprend de l'ubiquinone-10.

13. Préparation pour la repousse des cheveux selon l'une des revendications précédentes, **caractérisée en ce que** la préparation pour la repousse des cheveux comprend des acides gras oméga-3 qui sont ajoutés à une composition pulvérulente de la préparation pour la repousse des cheveux sous forme de microcapsules qui contiennent les acides gras oméga-3.

14. Préparation pour la repousse des cheveux selon l'une des revendications précédentes, **caractérisée en ce que** la préparation pour la repousse des cheveux comprend des caroténoïdes naturels, des rétinoïdes, de la thiamine, de la riboflavine, du chrome, de l'iode, du manganèse, du molybdène, du vanadium, du calcium, du potassium, de l'acide a-lipoïque, de la choline, de l'inositol, de la L-carnitine, de l'acide p-aminobenzoïque, de l'acide gamma-aminobutyrique, de la sylimarine et des les bioflavonoïdes d'agrumes.

15. Procédé pour personnaliser une préparation pour la repousse des cheveux avec au moins une composition de substances adaptables selon la revendication 1 ou selon l'une des revendications précédentes 2 à 14, **caractérisé en ce que** les étapes suivantes du procédé sont réalisées:
a) - fournir une version de base du préparation pour la repousse des cheveux pour femmes avec 20,0 mg de zinc et 2,5 mg de fer;
b) - fournir une version de base de la préparation pour la repousse des cheveux pour hommes avec 25,0 mg de zinc et 1,0 mg de fer;
c) - demander à une cliente ou un client de répondre à des questionnaires;
d) - effectuer des analyses de sang, d'urine, de créatinine, de salive, de cheveux, de selles et d'haleine;
e) - déterminer, à l'aide des évaluations du questionnaire et des analyses de sang, d'urine, de créatinine, de salive, de cheveux, de selles et d'haleine, les substances différentes et les dosages différents par rapport aux dosages de la version de base;
f) - sélection de la version de base du préparation pour la repousse des cheveux pour femmes dans le cas d'une cliente;
g) - sélection de la version de base du préparation pour la repousse des cheveux pour hommes dans le cas d'un client;
h) - adaptation de la version de base sélectionnée de la préparation pour la repousse des cheveux aux besoins individuels déterminés en matière d'apport en nutriments organiques d'une cliente ou d'un client, en ajoutant les substances différentielles déterminées et les dosages différentiels aux dosages de la version de base sélectionnée.

16. Mélange de substances contenant les substances L-cystéine et zinc, destiné à être utilisé en thérapie contre la chute des cheveux et pour la stimulation de la repousse des cheveux, **caractérisé en ce que** le mélange de substances contient 1'000,0 mg à 2'000,0 mg de L-cystéine **et que** le mélange de substances contient également du cuivre, du calcium, du magnésium, du silicium, du fer et du cholécalciférol, **ainsi que** de la gomme de guar ou de la gomme de caroube pour une absorption retardée dans l'intestin grêle **et qu**'une version de base du mélange de substances pour femmes avec 20,0 mg de zinc et 2,5 mg de fer ou une version de base du mélange de substances pour hommes avec 25,0 mg de zinc et 1,0 mg de fer sont sélectionnables **et que** les substances constitutives ou compositions de substances de la version de base sélectionnée sont adaptées ou sont complétées sur la base d'évaluations par questionnaire et de tests de sang, d'urine, de créatinine, de salive, de cheveux, de selles et d'haleine apport individuel déterminé de nutriments organiques d'une cliente ou d'un client.

17. Mélange de substances à utiliser selon la revendication 16, **caractérisé en ce que** le mélange de substances contient de la vitamine E naturelle sous forme d'alpha-tocophérol et de gamma-tocophérol.

18. Mélange de substances à utiliser selon la revendication 16 ou 17, **caractérisé en ce que** le mélange de substances contient de l'acide ascorbique.

19. Mélange de substances à utiliser selon l'une des revendications précédentes 17-18, **caractérisé en ce que** le mélange de substances contient de la biotine.

20. Mélange de substances à utiliser selon l'une des revendications précédentes 17 à 19, **caractérisé en ce que** le mélange de substances contient de la L-méthionine.

21. Mélange de substances à utiliser selon l'une des revendications précédentes 17 à 20, **caractérisé en ce que** le mélange de substances contient de l'acide pantothénique.

22. Mélange de substances à utiliser selon l'une des revendications précédentes 17 à 21, **caractérisé en ce que** le mélange de substances contient de la pyridoxine et/ou du pyridoxal et/ou de la pyridoxamine.

23. Mélange de substances à utiliser selon l'une des revendications précédentes 17 à 22, **caractérisé en ce que** le mélange de substances contient de la cobalamine.

24. Mélange de substances à utiliser selon l'une des revendications précédentes 17 à 23, **caractérisé en ce que** le mélange de substances contient du sélénium.

25. Mélange de substances à utiliser selon l'une des revendications précédentes 17 à 24, **caractérisé en ce que** le mélange de substances contient de l'acide nicotinique.

26. Mélange de substances à utiliser selon l'une des revendications précédentes 17 à 25, **caractérisé en ce que** le mélange de substances contient de l'acide folique.

27. Mélange de substances à utiliser selon l'une des revendications précédentes 17 à 26, **caractérisé en ce que** le mélange de substances contient de l'ubiquinone-10.

28. Mélange de substances à utiliser selon l'une des revendications précédentes 17 à 27, **caractérisé en ce que** le mélange de substances contient des acides gras oméga-3 qui sont ajoutés à une composition pulvérulente du mélange de substances sous forme de microcapsules qui contiennent les acides gras oméga-3.

29. Mélange de substances à utiliser selon l'une des revendications précédentes 17 à 28, **caractérisé en ce que** le mélange de substances contient des caroténoïdes naturels, des rétinoïdes, de la thiamine, de la riboflavine, du chrome, de l'iode, du manganèse, du molybdène, du vanadium, du calcium, du potassium, de l'acide a-lipoïque, de la choline, del'inositol, de la L-carnitine, de l'acide p-aminobenzoïque, de l'acide gamma-aminobutyrique, de la sylimarine et des les bioflavonoïdes d'agrumes.
